# EUROPÄISCHE PATENTSCHRIFT

(11) **EP 3 478 335 B1**
(45) Veröffentlichungstag und Bekanntmachung des Hinweises auf die Patenterteilung: **12.08.2020**
(21) Anmeldenummer: 17733837.3
(22) Anmeldetag: 28.06.2017
(51) Int. Cl.: A61M 1/16, A61M 1/34

(54) **BLUTBEHANDLUNGSVORRICHTUNG ZUR BLUTERKENNUNG IN EINER DIALYSIERFLÜSSIGKEITSABFÜHRLEITUNG**
BLOOD TREATMENT DEVICE FOR BLOOD DETECTION IN A DIALYSIS FLUID DISCHARGE LINE
DISPOSITIF DE TRAITEMENT DU SANG POUR DÉTECTER DU SANG DANS UNE LIGNE D'ÉVACUATION DU LIQUIDE DE DIALYSE

(30) Priorität: 29.06.2016 DE 102016007828
(43) Veröffentlichungstag der Anmeldung: 08.05.2019
(73) Patentinhaber: Fresenius Medical Care Deutschland GmbH, 61352 Bad Homburg (DE)
(72) Erfinder: GAGEL, Alfred, 96123 Litzendorf (DE)
(86) Internationale Anmeldenummer: PCT/EP2017/066039
(87) Internationale Veröffentlichungsnummer: WO 2018/002162

(56) Entgegenhaltungen:
- EP-A1- 0 623 357
- US-A1- 2011 089 112
- US-A1- 2013 313 194

## Beschreibung

Die Erfindung betrifft eine Blutbehandlungsvorrichtung zur Bluterkennung in einer Dialysierflüssigkeitsabführleitung der Blutbehandlungsvorrichtung.

Bei Verfahren der chronischen Blutreinigungstherapie wird Blut über einen extrakorporalen Blutkreislauf geleitet. Bei der Hämodialyse (HD) wird das Blut durch einen Dialysator gereinigt, der eine im extrakorporalen Blutkreislauf befindliche Blutkammer und eine Dialysierflüssigkeitskammer aufweist, die durch eine semipermeable Membran voneinander getrennt sind. Die Dialysierflüssigkeitskammer wird während einer Hämodialysebehandlung von Dialysierflüssigkeit durchflossen, wobei bestimmte Substanzen aufgrund der Diffusion zwischen dem Blut und der Dialysierflüssigkeit durch die Membran transportiert und mit der Dialysierflüssigkeit über einen Dialysierflüssigkeitskreislauf entfernt werden. Bei der Hämofiltration (HF) werden bestimmte Substanzen aufgrund von Konvektion durch eine Filtermembran aus dem Blut gefiltert. Die Hämodiafiltration (HDF) stellt eine Kombination aus beiden Verfahren dar.

Dem Patienten bei den Blutreinigungsverfahren entzogene Flüssigkeit lässt sich durch eine Substitutionsflüssigkeit ersetzen, die dem extrakorporalen Blutkreislauf während der Blutbehandlung zugeführt wird.

Im Dialysierflüssigkeitskreislauf befindet sich eine Dialysierflüssigkeitspumpe zur Förderung der Dialysierflüssigkeit. Eine Ultrafiltrationspumpe erzeugt den nötigen Unterdruck in der Dialysierflüssigkeitskammer des Dialysators, damit dem Patienten zur Erzielung des gewünschten Flüssigkeitshaushalts Flüssigkeit entfernt werden kann, die nicht durch Substitutionsflüssigkeit ersetzt wird.

Die Membran besteht im allgemeinen aus Hohlfasern. Die Integrität der Membran gewährleistet eine Trennung des Bluts von der Dialysierflüssigkeit.

Zum Schutz des Patienten weisen die bekannten Dialysegeräte verschiedene Sicherheitssysteme auf. Ein bekanntes Sicherheitssystem umfasst einen Blutleckdetektor, der die Membran des Dialysators auf Leckstellen überwacht, die dazu führen, dass Blut aus dem Blutkreislauf in den Dialysierflüssigkeitskreislauf gelangt. Die Detektion von Blut in der Dialysierflüssigkeit erfolgt bei den bekannten Blutleckdetektoren im allgemeinen mittels eines foto-optischen Sensors, mit dem sich die Konzentration von Blut in der an sich klaren Dialysierflüssigkeit bestimmen lässt. Die Blutleckdetektoren werden vielfach im Durchfluss betrieben und erzeugen ein Signal, wenn ein vorgegebener Grenzwert überschritten wird. Im Falle eines Blutlecks, bei dem in der Folge ein vorgegebener Grenzwert überschritten wird, können verschiedene Maßnahmen eingeleitet werden. Es kann ein Alarm ausgelöst werden und die Stromversorgung der Blutpumpe kann unterbrochen werden. In einigen Systemen wird auch die Dialysatseite des Dialysators mittels einer Bypass-Leitung überbrückt.
Eine Dialysierflüssigkeitszuführleitung ist in diesem Kurzschlussfall direkt mit einer Dialysierflüssigkeitsabführleitung verbunden, die im Normalbetrieb zuerst in die Dialysierflüssigkeitskammer mündet. Diese Überbrückung des Dialysators ist durch entsprechende Ansteuerung von beispielsweise Klemmen, Ventilen oder anderen Unterbrechungseinrichtungen in der Dialysierflüssigkeitszuführleitung, der Dialysierflüssigkeitsabführleitung und der Bypass-Leitung durchführbar.

Der Blutleckdetektor ist herkömmlich stromabwärts einer Stelle an der Dialysierflüssigkeitsabführleitung angeordnet, an der die Bypass-Leitung in die Dialysierflüssigkeitsabführleitung mündet.

Fließt die Dialysierflüssigkeit mit höherer Geschwindigkeit durch die Dialysierflüssigkeitsabführleitung, ist die absolute Blutkonzentration in der Dialysierflüssigkeitsabführleitung niedriger als bei langsamer Fließgeschwindigkeit. Die übertretende Blutmenge pro Zeit wird stärker verdünnt. Daher wird herkömmlicherweise eine Kopplung des Grenzwerts mit der Fließgeschwindigkeit derart durchgeführt, dass eine unerwünscht hohe Blutmenge durch die Membran tretende Blutmenge bei derselben absoluten Blutmenge in der Dialysierflüssigkeit erkannt wird. Der Grenzwert sinkt also beispielsweise umgekehrt proportional zu der Fließgeschwindigkeit, denn bei einer stärkeren Verdünnung des Bluts muss die Empfindlichkeit des Blutlecksensors erhöht werden.

Fließt durch entsprechende Ansteuerung von Klemmvorrichtungen im Dialysatkreislauf die Dialysierflüssigkeit nicht mehr über die Dialysierflüssigkeitskammer, sondern über die geöffnete Bypass-Leitung, ist die Hämodialyse gestoppt. Bei weiterhin stattfindender Ultrafiltration wird durch die Ultrafiltrationspumpe und den durch sie erzeugten Unterdruck in der Dialysierflüssigkeitskammer des Dialysators dem Patienten weiterhin Flüssigkeit entzogen, die ebenfalls durch die Dialysierflüssigkeitabführleitung fließt. Liegt in einem solchen Fall ein Blutleck im Dialysator vor, kann die relative Blutkonzentration in der Dialysierflüssigkeitabführleitung unmittelbar stromab des Dialysators stark ansteigen, da keine Dialysierflüssigkeit in den Dialysator nachströmt. Durch die geöffnete Bypass-Leitung wird aber weiterhin die Dialysierflüssigkeit mit derselben Geschwindigkeit wie vorher gepumpt und verdünnt die vom Dialysator kommende, im Fall eines Blutlecks stark mit Blut angereicherte Dialysierflüssigkeit. Im Blutlecksensor wird somit kein Blutleck erkannt, wenn die verdünnte Dialysierflüssigkeit keine Blutkonzentration oberhalb des Grenzwerts aufweist. In einem Extremfall ist eine starke Verfärbung der Dialysierflüssigkeit in der Dialysierflüssigkeitabführleitung zwischen Dialysator und der Stelle, an der die Dialysierflüssigkeitabführleitung in die Bypass-Leitung mündet, zu erkennen, die für den Patienten und das Pflegepersonal sichtbar ist. Dass dennoch kein Alarm erfolgt, kann fälschlicherweise als Ausfall oder Fehlfunktion des Blutleckdetektors interpretiert werden, sowie zu Verunsicherungen insbesondere des Patienten führen.

Bei der aus EP 0 623 357 bekannten Dialysevorrichtung ist ein Blutleckdetektor vorgesehen, dessen Empfindlichkeit automatisch an die Flussrate der Dialysierflüssigkeit angepasst wird. Aufgabe der vorliegenden Erfindung ist, ein Verfahren zur Bluterkennung in einer Dialysierflüssigkeitsabführleitung einer Blutbehandlungsvorrichtung, vorzuschlagen, das mit und ohne Bypass-Betrieb eine Blutgrenzwertüberschreitung stromabwärts des Dialysators erkennt, sowie eine Steuereinheit zu dessen Durchführung.

Die Aufgabe wird durch eine Blutbehandlungsvorrichtung mit den Merkmalen von Anspruch 1 gelöst. Vorteilhafte Ausführungsformen sind in den Unteransprüchen 2 bis 8 gekennzeichnet. Eine erfindungsgemäße Blutbehandlungsvorrichtung zur Erkennung einer Überschreitung eines ersten Grenzwerts einer ersten Blutkonzentration in einem ersten Abschnitt einer Dialysierflüssigkeitsabführleitung stromab einer Dialysierflüssigkeitskammer eines Dialysators der Blutreinigungsvorrichtung und stromauf eines Knotenpunkts, bei dem eine den Dialysator umgehende Bypass-Leitung in die Dialysierflüssigkeitsabführleitung mündet, wobei die Bypass-Leitung stromauf der Dialysierflüssigkeitskammer von einer Dialysierflüssigkeitszuführleitung abzweigt, die geeignet ist, der Dialysierflüssigkeitskammer von einer Dialysierflüssigkeitsquelle Dialysierflüssigkeit zuzuführen, besitzt einen Blutleckdetektor in einem zweiten Abschnitt der Dialysierflüssigkeitsabführleitung stromab des Knotenpunkts, der geeignet ist, ein Überschreiten eines zweiten Grenzwerts einer zweiten Blutkonzentration im zweiten Abschnitt stromab des Knotenpunkts zu erkennen und an die Steuereinheit zu melden, wobei der zweite Grenzwert mit steigender Fließgeschwindigkeit in der Dialysierflüssigkeitszuführleitung vor der Abzweigung der Bypass-Leitung abnimmt, und wobei die Steuereinheit konfiguriert ist, in einem Bypass-Betrieb, bei dem Dialysierflüssigkeit von der Dialysierflüssigkeitsquelle über die Dialysierflüssigkeitszuführleitung, die Bypass-Leitung und den zweiten Abschnitt der Dialysierflüssigkeitsabführleitung geführt wird, den ersten Grenzwert dann als überschritten anzusehen, wenn der Blutleckdetektor einen dritten Grenzwert als überschritten erkennt, der der bei der Fließgeschwindigkeit niedriger ist als der zweite Grenzwert. Die Fließgeschwindigkeit bezeichnet dabei diejenige Fließgeschwindigkeit, der der jeweilige zweite Grenzwert zugeordnet ist.

Die Erfindung hat den Vorteil, dass ohne zusätzlichen Blutleckdetektor im ersten Abschnitt stromab des Dialysators dort dennoch eine unerwünscht hohe Blutkonzentration festgestellt werden kann.
Im Bypass-Betrieb kann es nicht mehr zu Irritationen beim Patienten oder Bedienungspersonal kommen, wenn trotz erkennbarer Verfärbung im ersten Abschnitt kein Blutleckalarm ausgelöst wird.
Des Weiteren wird nicht fälschlicherweise auf einen Ausfall oder eine Fehlfunktion des Blutleckdetektors geschlossen, denn dieser erkennt tatsächlich eine zu hohe Blutkonzentration.

Nach einem Aspekt der Erfindung ist die Steuereinheit so konfiguriert, dass der dritte Grenzwert unabhängig von der Fließgeschwindigkeit der Dialysierflüssigkeit in der Dialysierflüssigkeitszuführleitung ist.

Dies ermöglicht eine einfach umzusetzende Grenzwertfestlegung.

Nach einem weiteren Aspekt der Erfindung ist die Steuereinheit derart konfiguriert, dass der dritte Grenzwert eine Konstante ist.

Dadurch ist vorteilhaft nur ein einziger Grenzwert zu speichern, was den Aufwand der Blutdetektion noch verringert.

Nach einer Variante der Erfindung ist die Steuereinheit konfiguriert, dass im Bypass-Betrieb eine Substitutionsrate einer Prädilution kleiner als ein Substitutionsratengrenzwert ist. Die Substitutionsrate bezieht sich auf eine Prädilution, wie sie im Bereich der Blutbehandlungsvorrichtungen bekannt ist. Dazu wird in einem extrakorporalen Blutkreislauf in Fließrichtung des Blutes gesehen stromauf der Blutkammer des Dialysators, in die eine arterielle Blutzuführleitung mündet, Substitutionsflüssigkeit zugeführt. Dies führt im normalen Betrieb dazu, dass das Blut im Dialysator verdünnt ist, bevor es durch eine Ultrafiltration im Dialysator wieder eingedickt wird. Ein Verringern oder gänzliches Stoppen oder Prädilution verringert den möglichen Fluss an Dialysierflüssigkeit im ersten Abschnitt der Dialysierflüssigkeitsabführleitung weiter und kann somit eine sichtbare Eintrübung des ersten Abschnitts der Dialysierflüssigkeitsabführleitung noch verstärken.

Nach dieser Variante ist vorteilhaft sichergestellt, dass auch bei dieser noch geringeren Verdünnung im ersten Abschnitt der Dialysierflüssigkeitsabführleitung eine Überschreitung des ersten Grenzwerts sicher erkannt wird.
Hierzu kann vorteilhafterweise der dritte Grenzwert bei verringerter oder gestoppter Prädilution noch niedriger als mit Prädilution angesetzt werden.

Die beschriebene Konfigurierung trifft grundsätzlich entsprechend für die Postdilution zu, bei der lediglich der Grad der Eintrübung ein anderer sein kann. Die Zufuhr der Substitutionsflüssigkeit findet in diesem Fall stromab der Blutkammer des Dialysators statt. Die Erfindung umfasst auch diese Variante.

Nach einem weiteren Aspekt der Erfindung ist die Steuereinheit konfiguriert, dass im Bypass-Betrieb ein Dialysatfluss durch den Dialysator kleiner als ein Dialysatdialysatorfluss-Grenzwert ist. Ein Bypass-Betrieb muss also nicht eine völlige Umgehung des Dialysators bedeuten, sondern es kann ein Restfluss durch die Dialysierflüssigkeitszuführleitung in die Dialysierflüssigkeitskammer des Dialysators verbleiben.

Die Festlegung eines Dialysatdialysatorfluss-Grenzwerts hat den Vorteil, dass bei einem geringen Restfluss durch den Dialysator die erfindungsgemäße Verwendung des niedrigen dritten Grenzwerts genutzt wird. Oberhalb des Dialysierflüssigkeitdialysatorfluss-Grenzwerts kann die Steuereinheit herkömmlich den zweiten, flussabhängigen Grenzwert verwenden, um bei dessen Überschreiten ein Blutleck zu erkennen.

Eine Variante der Erfindung sieht vor, dass die Steuereinheit konfiguriert ist, dass die Fließgeschwindigkeit durch eine Pumpeinrichtung in der Dialysierflüssigkeitszuführleitung vorgegeben ist.

Dies ermöglicht eine einfache Festlegung von Grenzwerten.

Insbesondere kann die Erfindung vorsehen, dass die Steuereinheit konfiguriert ist, dass die Fließgeschwindigkeit durch eine Bilanziereinrichtung vorgegeben ist.
Eine Bilanziereinrichtung, wie etwa ein Bilanzkammersystem, ist bekannt und muss hier nicht näher beschrieben werden. Einfach ausgedrückt lässt sich die die Menge an Dialysierflüssigkeit, die über die Dialysierflüssigkeitszuführleitung zugeführt wird, und die Menge an Ultrafiltrat sowie Dialysierflüssigkeit, die über Dialysierflüssigkeitszuführleitung abgeführt werden, gezielt aufeinander abstimmen. Die Fließgeschwindigkeit in der Dialysierflüssigkeitszuführleitung wird über eine Füllung und Leerung derjenigen Bilanzkammer der Bilanziereinrichtung, die mit dieser Dialysierflüssigkeitszuführleitung in Verbindung steht, bestimmt.

Wenn im Rahmen der Erfindung davon die Rede ist, dass die Steuereinheit oder eine andere geeignete Einheit etwas ausführt, ist dies eine vereinfachte Ausdrucksweise und dahingehend zu verstehen, dass die Steuereinheit oder die andere geeignete Einheit gegebenenfalls einen geeigneten Aktor oder Sensor ansteuert, etwas auszuführen, falls die Steuereinheit oder die andere geeignete Einheit nicht selbst zur direkten Ausführung in der Lage ist. Der Fachmann weiß in diesen Fällen, dass die entsprechende vereinfachte Formulierung entsprechend zu verstehen ist.

Als Unterbrechungsmittel können beispielsweise in einem extrakorporaten Blutkreislauf herkömmlich verwendete Unterbrechungsmittel, wie etwa Schlauchklemmen oder Ventile, von der Steuereinheit ansteuerbar sein.

Eine erfindungsgemäße Blutbehandlungsvorrichtung zur extrakorporalen Blutbehandlung, insbesondere zur Hämodiafiltration, ist vorzugsweise vorgesehen zur Aufnahme eines Blutleitungssystems eines extrakorporalen Blutkreislaufs, wobei das Blutleitungssystem einen arteriellen Abschnitt einer Blutzuführleitung und einen venösen Abschnitt einer Blutrückführleitung besitzt. Der arterielle Abschnitt und/oder der venöse Abschnitt sind dafür vorgesehen, mit einem Blutgefäß, insbesondere einer arteriovenösen Fistel, in Fluidverbindung gesetzt zu werden, und die Blutbehandlungsvorrichtung weist zumindest eine Druckerzeugungseinrichtung auf, die geeignet ist, auf den arteriellen und/oder den venösen Abschnitt einzuwirken.

Bei Verfahren der chronischen Blutreinigungstherapie wird Blut über einen extrakorporalen Blutkreislauf geleitet. Bei der Hämodialyse (HD) wird das Blut durch einen Dialysator gereinigt, der eine im extrakorporalen Blutkreislauf befindliche Blutkammer und eine Dialysierflüssigkeitskammer aufweist, die durch eine semipermeable Membran voneinander getrennt sind. Die Dialysierflüssigkeitskammer wird während einer Hämodialysebehandlung von Dialysierflüssigkeit durchflossen, wobei bestimmte Substanzen aufgrund der Diffusion zwischen dem Blut und der Dialysierflüssigkeit durch die Membran transportiert und mit der Dialysierflüssigkeit über einen Dialysierflüssigkeitskreislauf entfernt werden. Bei der Hämofiltration (HF) werden bestimmte Substanzen aufgrund von Konvektion durch eine Filtermembran aus dem Blut gefiltert. Die Hämodiafiltration (HDF) stellt eine Kombination aus beiden Verfahren dar.

Dem Patienten bei den Blutreinigungsverfahren entzogene Flüssigkeit rasst sich durch eine Substitutionsflüssigkeit ersetzen, die dem extrakorporalen Blutkreislauf während der Blutbehandlung zugeführt wird.

Im Dialysierflüssigkeitskreislauf befindet sich eine Dialysierflüssigkeitspumpe zur Förderung der Dialysierflüssigkeit. Eine Ultrafiltrationspumpe erzeugt den nötigen Unterdruck in der Dialysierflüssigkeitskammer des Dialysators, damit dem Patienten zur Erzielung des gewünschten Flüssigkeitshaushalts Flüssigkeit entfernt werden kann, die nicht durch Substitutionsflüssigkeit ersetzt wird.

Dabei wirkt die Ultrafiltrationspumpe im Betrieb herkömmlich auf den extrakorporalen Blutkreislauf dadurch ein, dass über eine semipermeable Membran eines Dialysators, die eine Blutkammer von einer Dialysierflüssigkeitskammer trennt, eine Konvektion zur Dialysierflüssigkeitskammer hin entsteht. Diese Konvektion wirkt als Unterdruck auf die Blutkammer des Dialysators und damit den mit der Blutkammer verbundenen extrakorporalen Blutkreislauf ein. Durch ein Ansteuern und Abschalten bzw. Anhalten der Ultrafiltrationspumpe wird ebenfalls eine Druckerzeugung, die auf den zumindest einen Abschnitt einwirken kann, beendet. Wie die jeweiligen Pumpen im Dialysierflüssigkeitskreislauf, im Blutleitungssystem und/oder im Substituatleitungssystem bezüglich der Leitung, in die sie eingebracht sind, und ihrer genauen Funktionalität bei einer Blutbehandlung vorliegen, hat keinen Einfluss auf das Prinzip der Erfindung. Entsprechend sind die Schilderungen zur herkömmlichen Nutzung der Ultrafiltrationspumpe nicht einschränkend, sondern nur beispielhaft zu sehen. So kann beispielsweise die Ultrafiltrationspumpe auch nur den Teil der Ultrafiltration bewirken, der nicht von einer Substitutionsrate kompensiert wird.

Des Weiteren weist die erfindungsgemäße Blutbehandlungsvorrichtung eine erfindungsgemäße Steuereinheit zur Erkennung einer Überschreitung eines ersten Grenzwerts einer ersten Blutkonzentration in einem ersten Abschnitt einer Dialysierflüssigkeitsabführleitung stromab der Dialysierflüssigkeitskammer des Dialysators der Blutbehandlungsvorrichtung und stromauf des Knotenpunkts, an dem die den Dialysator umgehende Bypass-Leitung in die Dialysierflüssigkeitsabführleitung mündet, wobei die Bypass-Leitung stromauf der Dialysierflüssigkeitskammer von einer Dialysierflüssigkeitszuführleitung abzweigt, die geeignet ist, der Dialysierflüssigkeitskammer von einer Dialysierflüssigkeitsquelle Dialysierflüssigkeit zuzuführen, auf.

Bei Vorliegen eines vorbestimmten Kriteriums kann die Steuereinheit oder die Auswerteinheit eine Meldung abgeben. Diese Meldung kann beispielsweise über einen Signalgeber erfolgen. Entsprechend kann die Steuereinheit und/oder Auswerteinheit zum Geben der Meldung konfiguriert sein.

Im Folgenden werden die Erfindung und weitere vorteilhafte Varianten und Ausführungsformen anhand von Ausführungsbeispielen unter Bezugnahme auf die Figur näher beschrieben.

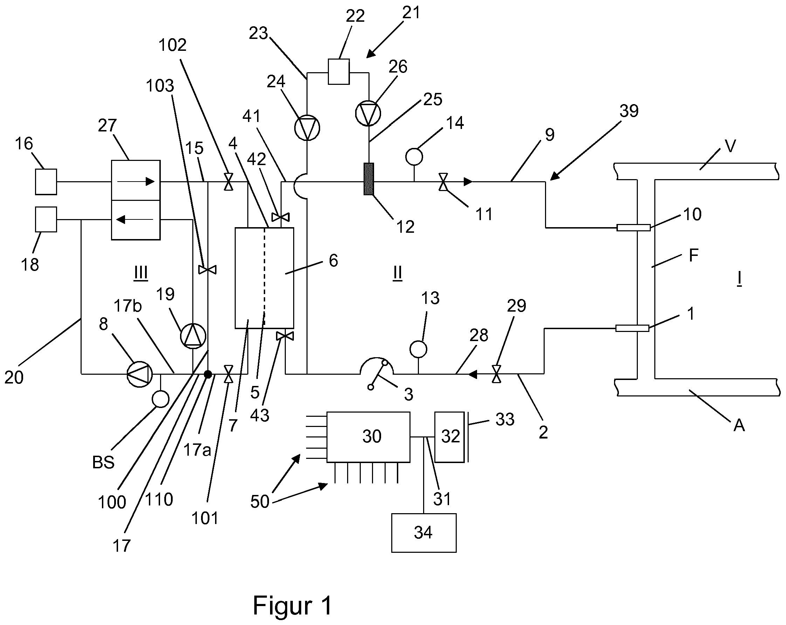

Die einzige Figur 1 zeigt schematisch den Aufbau einer Hämodiafiltrationsvorrichtung (HDF-Vorrichtung) zusammen mit einer erfindungsgemäßen Steuereinheit zur Erkennung einer Überschreitung eines ersten Grenzwerts einer ersten Blutkonzentration in einem ersten Abschnitt einer Dialysierflüssigkeitsabführleitung stromab einer Dialysierflüssigkeitskammer eines Dialysators.

Anhand der Figur werden zunächst der prinzipielle Aufbau eines Hämodiafiltrationsgeräts und seine Anbindung an das lediglich angedeutete Blutgefäßsystem I eines (nicht dargestellten) Patienten kurz erläutert. Bei der Hämodialyse wird Blut aus dem Blutgefäßsystem I in einen extrakorporalen Blutkreislauf II geleitet. Zu diesem Zweck ist dem Patienten eine Fistel F gelegt, die einen Kurzschluss zwischen einer Arterie A und einer Vene V beispielsweise im linken Unterarm (nicht dargestellt) bildet und somit eine sogenannte arteriovenöse Fistel darstellt. Über eine arterielle Kanüle 1 ist eine Blutzuführleitung 2 mit der Fistel F verbunden. Blut aus dem Blutgefäßsystem I wird mittels einer Blutpumpe 3 über die Blutzuführleitung 2 einem als Hämodialysator 4 ausgeführten Blutreinigungselement zugeführt. Im Hämodialysator 4 trennt eine vorzugsweise in Form einer Vielzahl von nicht dargestellten Hohlfasern ausgebildeten semipermeablen Membran 5 eine auch als Blutkammer bezeichnete erste Kammer 6, die Teil des extrakorporalen Blutkreislaufs II ist, von einer auch als Dialysierflüssigkeitskammer bezeichneten zweiten Kammer 7, die Teil eines Dialysierflüssigkeitskreislaufs III ist. Durch die semipermeable Membran 5 treten aus dem Blut zu entfernende Stoffe in die Dialysierflüssigkeit über, die durch die Dialysierflüssigkeit abgeführt werden. Gleichzeitig kann über einen Druckgradienten eine überschüssige Flüssigkeitsmenge aus dem Blut ultrafiltriert werden und ebenfalls über die abfließende Dialysierflüssigkeit entfernt werden. Der Druckgradient wird durch eine Ultrafiltrationspumpe 8 erzeugt.

Das gereinigte Blut verlässt die Blutkammer 6 des Hämodialysators 4 über eine Blutrückführleitung 9 und wird über eine venöse Kanüle 10, die in einen der Vene V des Patienten zugewandten Teil der Fistel F eingestochen ist, in das Blutgefäßsystem I des Patienten zurückgeführt. An der Blutrückführleitung 9 ist eine venöse Klemme 11 als venöse Unterbrechungseinrichtung vorgesehen, mit der die Rückführung von Blut beispielsweise in Notfällen unterbrochen werden kann. Derartige Notfälle können z.B. auftreten, wenn durch einen Luftdetektor 12 zwischen Dialysator 4 und venöser Klemme 11 Luft in der Blutrückführleitung 9 detektiert wird. An der Blutzuführleitung 2 ist stromauf der Blutpumpe 3 ein arterieller Drucksensor 13 und an der Blutrückführleitung 9 stromauf der venösen Klemme 11 ein venöser Drucksensor 14 vorgesehen.

Die zweite Kammer 7 des Hämodialysators 4 wird von Dialysierflüssigkeit durchströmt, die über eine Dialysierflüssigkeitszuführleitung 15 von einer Dialysierflüssigkeitsquelle 16 zugeführt und über eine Dialysierflüssigkeitsabführleitung 17 zu einem Abfluss 18 abgeführt wird. Die Dialysierflüssigkeit wird durch eine Dialysierflüssigkeitspumpe 19 in der Dialysierflüssigkeitsabführleitung 17 gefördert. Stromauf der Dialysierflüssigkeitspumpe 19 zweigt von der Dialysierflüssigkeitsabführleitung 17 eine Ultrafiltratleitung 20 ab, in die die Ultrafiltrationspumpe 8 geschaltet ist und die auch zum Abfluss 18 führt.

Um dem Patienten wieder Flüssigkeit zuzuführen, verfügt das HDF-Gerät über eine Substitutionseinrichtung 21, mit der dem Blut im extrakorporalen Blutkreislauf II eine Substitutionsflüssigkeit (auch als Substituat bezeichnet) zugeführt werden kann. Die Substitutionseinrichtung 21 weist eine Substituatquelle 22 zur Bereitstellung von Substituat auf, von der eine erste Substituatleitung 23, in die eine erste Substituatpumpe 24 geschaltet ist, stromab der Blutpumpe 3 in die Blutzuführleitung 2 führt, was als Prädilution bezeichnet wird, da die Substituatzuführung vor der Blutkammer 6 erfolgt. Von der Substituatquelle 22 führt eine zweite Substituatleitung 25, in die eine zweite Substituatpumpe 26 geschaltet ist, stromab der Blutkammer 6 (Postdilution) in die Blutrückführleitung 9. Die zweite Substituatleitung 25 mündet in die Tropfkammer 12 der Rückführleitung 9.

Verschiedene Bilanziereinrichtungen ermöglichen, die Menge an Substituat und Dialysierflüssigkeit, die zugeführt werden, und die Menge an Ultrafiltrat sowie Dialysierflüssigkeit, die abgeführt werden, im Zusammenspiel mit den genannten und eventuell zusätzlichen Pumpen gezielt aufeinander abzustimmen. Dem Fachmann stehen zur Realisierung einer Bilanziereinrichtung 27, die zugeführte Dialysierflüssigkeit und abgeführte Dialysierflüssigkeit bilanziert, und eventueller weiterer Bilanziereinrichtungen und Pumpen im Dialysierflüssigkeitskreislauf und bei der Substitutionseinrichtung verschiedenste Ausgestaltungen zur Verfügung, so dass an dieser Stelle von detaillierten Ausführungen abgesehen wird. Gleiches gilt auch für die Bereitstellung von Dialysierflüssigkeit durch die Dialysierflüssigkeitsquelle 16 und von Substituat durch die Substituatquelle 22.

Auch zum Einsatz von Aktoren und Sensoren in einem HDF-Gerät im Allgemeinen stehen dem Fachmann zahlreiche Möglichkeiten zur Verfügung, ohne dass hier im Detail auf alle diese Möglichkeiten eingegangen werden muss. Die Darstellung in der Figur ist auf einige wenige dieser Aktoren und Sensoren beschränkt, die für die Erläuterung der Erfindung ausreichend sind, wie etwa die Klemme 103 in der Bypass-Leitung 100, den Blutleckdetektor BS und die Ultrafiltrationspumpe 8.

Das HDF-Gerät wird durch eine Steuereinheit 30 gesteuert und überwacht. Hierzu ist die Steuereinheit 30 mit den einzelnen Aktoren und Sensoren des Gerätes mit Signalleitungen verbunden. Für die in der Figur dargestellten Aktoren und Sensoren, wie etwa Pumpen, Drucksensoren, Klemmen, Ventile und Blutleckdetektoren, ist dies durch eine Mehrzahl von Signalleitungen 50 lediglich pauschal angedeutet, die für die einzelnen Aktoren oder Sensoren bzw. Detektoren wegen der sich sonst ergebenden schlechten Übersichtlichkeit nicht einzeln dargestellt und nicht mit einem individuellen Bezugszeichen bezeichnet sind.

Die erfindungsgemäße Steuereinheit zur Bestimmung des Drucks in einem Blutgefäß wird im Zusammenspiel mit der eben beschriebenen Hämodiafiltrationsvorrichtung erläutert, da in dieser die meisten oder sogar alle gemäß der Erfindung angesteuerten Hardware-Komponenten, insbesondere Aktoren und Sensoren, bereits vorhanden sind. Die Erfindung beschränkt sich aber nicht auf die Anwendung der Steuereinheit in der konkret beschriebenen HDF-Vorrichtung. Die Steuereinheit kann Bestandteil des HDF-Geräts sein oder eine separate Einheit bilden, die an ein bestehendes HDF-Gerät anzuschließen ist. Entsprechendes gilt aber für jede andere Blutbehandlungsvorrichtung, wie etwa ein Hämofiltrationsgerät und ein Hämodialysegerät, an die eine erfindungsgemäße Steuereinheit angeschlossen werden kann.

Die im Folgenden als von der Steuereinheit ausgeführt erläuterten Verfahrensschritte können des Weiteren entweder allesamt von der erfindungsgemäßen Steuereinheit gesteuert werden oder im Rahmen des erfindungsgemäßen Verfahrens wahlweise zumindest teilweise manuell ausgeführt werden, bzw. von weiteren Einrichtungen, wie einer Auswerteinheit, einer Speichereinheit, einer Eingabeeinheit, einem Signalgeber oder weiteren Einrichtungen, die ihrerseits Schritte nach Ansteuerung durch die Steuereinheit oder manuell bedient oder selbstständig ausführen, ausgeführt werden.

Wenn im Folgenden davon die Rede ist, dass die Steuereinheit oder eine andere geeignete Einheit etwas "ausführt", beispielsweise eine Blutkonzentration misst oder eine Klemme schließt, ist dies eine vereinfachte Ausdruckweise und dahingehend zu verstehen, dass die Steuereinheit oder die andere geeignete Einheit einen geeigneten Aktor oder Sensor gegebenenfalls nach Abfrage eines Status ansteuert, etwas auszuführen, beispielsweise einen Blutdetektor ansteuert, eine Blutkonzentration zu messen und die gemessene Blutkonzentration an die Steuereinheit zu melden, oder eine Klemme ansteuert, zu schließen, eventuell nach Abfrage, ob diese bereits geschlossen ist, etc. Der Einfachheit halber wird nicht in allen Fällen ausformuliert, welcher Aktor oder Sensor nach einer Ansteuerung aktiv wird. Der Fachmann weiß in diesen Fällen, wie die entsprechende vereinfachte Formulierung zu verstehen ist.

Die weiteren Konfigurationen der Steuereinheit werden im Folgenden im Rahmen des erfindungsgemäßen Verfahrens geschildert.

Gemäß dem Ausführungsbeispiel des erfindungsgemäßen Verfahrens befindet sich der Patient zunächst in einem laufenden Hämodiafiltrationsverfahren. Das heißt, die Blutpumpe 3 pumpt Blut aus der Fistel F durch die Blutzuführleitung 2 in die erste Kammer 6 des Hämodialysators 4 und über die Blutrückführleitung 9 und die venöse Kanüle 10 zurück in die Fistel F. Die venöse Klemme 11 und die arterielle Klemme 29 in der Blutzuführleitung als arterielle Unterbrechungseinrichtung sind geöffnet. Durch den Hämodialysator 4 werden dem Blut unerwünschte Bestandteile entzogen und das Blut dadurch gereinigt.

Durch die Bilanziervorrichtung 27 ist ein Fluss Q1 in der Dialysierflüssigkeitszuführleitung eingestellt. Der Blutleckdetektor misst die zweite Blutkonzentration B2 im zweiten Abschnitt 17b der Dialysierflüssigkeitsabführleitung 17 stromab des Knotenpunkts 110, an dem die Bypass-Leitung 100 in die Dialysierflüssigkeitsabführleitung 17 mündet und meldet den Wert der zweiten Blutkonzentration B2 der Steuereinheit 30. In Abhängigkeit der Fließgeschwindigkeit Q1 liegt der Steuereinheit ein zweiter Grenzwert G2 vor. Falls beispielsweise durch eine Membranruptur Blut durch die Membran 5 in die Dialysierflüssigkeitskammer 7 gelangt, wird dieses in der entsprechenden Konzentration über die Dialysierflüssigkeitsabführleitung 17 bis zum Blutleckdetektor BS gepumpt. Wird dort eine zweite Blutkonzentration B2 gemessen, die höher ist als der Grenzwert G2, erkennt dies die Steuereinheit 30. Nun gibt die Steuereinheit 30 beispielsweise ein Alarmsignal und/oder stoppt die Blutpumpe 3 oder nimmt sonst eine Reaktion vor. Dies können übliche Maßnahmen, wie eine optische Anzeige, sein.

In Rahmen des Ausführungsbeispiels wird während des Dialysebetriebs auf einen Bypass-Betrieb umgeschaltet. Dazu werden die Klemmen 101 und 102 geschlossen und die Bypass-Leitung 100 durch Öffnen des Ventils 103 geöffnet. Die Dialysierflüssigkeit wird nun bei einer weiter arbeitenden Bilanzierkammer 27 mit demselben Fluss wie bisher über die Bypass-Leitung 100 gepumpt. Die Dialysierflüssigkeit fließt jedoch nicht mehr durch die Dialysierflüssigkeitskammer 7, so dass keine Dialyse durch Diffusion stattfindet. Wegen der weiterhin laufenden Ultrafiltrationspumpe 8 wird jedoch noch weiterhin eine Hämofiltration betrieben. Es tritt Plasmaflüssigkeit von der Blutkammer 6 über die Membran 5 in die Dialysierflüssigkeitskammer 7 über und fließt über die die Dialysierflüssigkeitsabführleitung 17 Richtung Abfluss 18. Bereits bei einer mittleren Ruptur kann eine starke Verfärbung im ersten Abschnitt 17a der Dialysierflüssigkeitsabführleitung 17a auftreten, da wegen der geringen Strömung, die lediglich durch die UF-Pumpe 8 bewirkt wird, nur eine geringe Verdünnung des Bluts stattfindet. Bei der Umschaltung in den Bypass-Betrieb hat die Steuereinheit als Grenzwert für einen Blutleckalarm einen hinterlegten dritten Grenzwert G3 übernommen, der in diesem Beispiel eine Konstante ist, die deutlich niedriger als der zweite Grenzwert G2 ist, der beim vorliegenden Fluss Q1 vorliegt, wie beispielsweise halb so groß wie der zweite Grenzwert G2. Durch diese stark erhöhte Empfindlichkeit löst die Steuereinheit somit einen Alarm aus, wenn zwar durch die Verdünnung der über die Bypass-Leitung 100 nachgelieferten Dialysierflüssigkeit die Blutkonzentration noch weit unter dem zweiten Grenzwert G2 liegt, aber trotzdem eine für Patienten und Bedienpersonal deutlich sichtbare Verfärbung der Dialysierflüssigkeit im ersten Abschnitt 17a der Dialysierflüssigkeitsabführleitung 17 auftritt.

In Rahmen eines weiteren Ausführungsbeispiels wird während des Dialysebetriebs auf einen Bypass-Betrieb umgeschaltet. Dazu wird die Klemmen 102 geschlossen, die Klemme 101 geöffnet und die Bypass-Leitung 100 durch Öffnen des Ventils 103 geöffnet. Die Dialysierflüssigkeit wird nun bei einer weiter arbeitenden Bilanzierkammer 27 mit demselben Fluss wie bisher über die Bypass-Leitung 100 gepumpt. Die Dialysierflüssigkeit fließt jedoch nicht mehr durch die Dialysierflüssigkeitskammer 7, so dass keine Dialyse durch Diffusion stattfindet. Wegen der weiterhin laufenden Ultrafiltrationspumpe 8 wird jedoch noch weiterhin eine Hämofiltration betrieben. Es tritt Plasmaflüssigkeit von der Blutkammer 6 über die Membran 5 in die Dialysierflüssigkeitskammer 7 über und fließt über die Dialysierflüssigkeitsabführleitung 17 Richtung Abfluss 18. Bereits bei einer mittleren Ruptur kann eine starke Verfärbung im ersten Abschnitt 17a der Dialysierflüssigkeitsabführleitung 17a auftreten, da wegen der geringen Strömung, die lediglich durch die UF-Pumpe 8 bewirkt wird, nur eine geringe Verdünnung des Bluts stattfindet. Bei der Umschaltung in den Bypass-Betrieb hat die Steuereinheit als Grenzwert für einen Blutleckalarm einen hinterlegten dritten Grenzwert G3 übernommen, der in diesem Beispiel eine Konstante ist, die deutlich niedriger als der zweite Grenzwert G2 ist, der beim vorliegenden Fluss Q1 vorliegt, wie beispielsweise halb so groß wie der zweite Grenzwert G2. Durch diese stark erhöhte Empfindlichkeit löst die Steuereinheit somit einen Alarm aus, wenn zwar durch die Verdünnung der über die Bypass-Leitung 100 nachgelieferten Dialysierflüssigkeit die Blutkonzentration noch weit unter dem zweiten Grenzwert G2 liegt, aber trotzdem eine für Patienten und Bedienpersonal deutlich sichtbare Verfärbung der Dialysierflüssigkeit im ersten Abschnitt 17a der Dialysierflüssigkeitsabführleitung 17 auftritt.

In einer Variante der Ausführungsbeispiele ist die Steuereinheit 30 konfiguriert, im Bypass-Betrieb die Prädilutionspumpe 24 anzusteuern und anzuhalten. Somit findet auch keine Verdünnung der Dialysierflüssigkeit in der Dialysierflüssigkeitsabführleitung 17 durch Substituat statt. In diesem Fall kann ein weiterer Grenzwert G3' vorgesehen bzw. hinterlegt sein, der noch niedriger ist, wie etwa zwei Drittel von G3. Je geringer die Verdünnung der Dialysierflussigkeit in der Dialysierflüssigkeitsabführleitung 17, desto niedriger kann der Grenzwert G3 angenommen werden.

Die Erfindung ist nicht auf die beschriebenen Ausführungsbeispiele beschränkt. Insbesondere kann jede Art von Blutleckdetektor, der Blut oder Blutbestandteile, wie insbesondere Hämatokrit, detektiert, gemäß der Erfindung zum Einsatz kommen. Außerdem können alle genannten Merkmale beliebig miteinander kombiniert werden, solange dies im Rahmen der Erfindung sinnvoll und machbar ist. Einzelne Schritte oder Unterschritte des Verfahrens können alle von der Steuereinheit ausgeführt werden.

Die Steuereinheit muss nicht immer dieselbe, einzige Steuereinheit sein. Es kann vielmehr eine erste Steueruntereinheit die für die Blutleckerkennung notwendigen Schritte durchführen, wie Grenzwertabrufen, Erkennen der Grenzwertüberschreitung, Veranlassen eines Alarms etc. Eine zweite Steueruntereinheit kann die restlichen Abläufe steuern, die für die Blutbehandlung notwendig sind. Dies kann die Ansteuerung von Aktoren und Sensoren sein etc. Auch andere Untereinheiten, wie Speichereinheiten, Darstellungseinheiten, Recheneinheiten etc. sollen unter dem Begriff Steuereinheit subsummiert sein.

## Patentansprüche

1. Blutbehandlungsvorrichtung zur extrakorporalen Blutbehandlung, insbesondere zur Hämodiafiltration, mit einer Steuereinheit (30) zur Erkennung einer Überschreitung eines ersten Grenzwerts (G1) einer ersten Blutkonzentration (B1) in einem ersten Abschnitt (17a) einer Dialysierflüssigkeitsabführleitung (17) stromab einer Dialysierflüssigkeitskammer (7) eines Dialysators (4) der Blutbehandlungsvorrichtung und stromauf eines Knotenpunkts (110), an dem eine den Dialysator (4) umgehende Bypass-Leitung (100) in die Dialysierflüssigkeitsabführleitung (17) mündet, wobei die Bypass-Leitung (100) stromauf der Dialysierflüssigkeitskammer (7) von einer Dialysierflüssigkeitszuführleitung (15) abzweigt, die geeignet ist, der Dialysierflüssigkeitskammer (7) von einer Dialysierflüssigkeitsquelle (16) Dialysierflüssigkeit zuzuführen,
wobei ein Blutleckdetektor (BS) in einem zweiten Abschnitt (17b) der Dialysierflüssigkeitsabführleitung (17) stromab des Knotenpunkts (110) vorgesehen ist, der geeignet ist, eine zweite Blutkonzentration (B2) im zweiten Abschnitt (17b) stromab des Knotenpunkts (110) zu messen, wobei die Steuereinheit konfiguriert ist ein Überschreiten eines zweiten Grenzwerts (G2) der zweiten Blutkonzentration (B2) zu erkennen, wobei der zweite Grenzwert (G2) mit steigender Fließgeschwindigkeit (Q1) in der Dialysierflüssigkeitszuführleitung (15) vor der Abzweigung der Bypass-Leitung (100) abnimmt und wobei die Steuereinheit (30) konfiguriert ist, in einem Bypass-Betrieb, bei dem Dialysierflüssigkeit von der Dialysierflüssigkeitsquelle (16) über die Dialysierflüssigkeitszuführleitung (15), die Bypass-Leitung (100) und den zweiten Abschnitt (17b) der Dialysierflüssigkeitsabführleitung (17) zu einem Abfluss (18) geführt wird, den ersten Grenzwert (G1) dann als überschritten anzusehen, wenn die zweite Blutkonzentration (B2) einen dritten Grenzwert (G3) überschreitet, der bei der Fließgeschwindigkeit (Q1) niedriger ist als der zweite Grenzwert (G2).

2. Blutbehandlungsvorrichtung nach Anspruch 1, **dadurch gekennzeichnet, dass** die Steuereinheit (30) konfiguriert ist, dass der dritte Grenzwert (G3) unabhängig von der Fließgeschwindigkeit (Q1) ist.

3. Blutbehandlungsvorrichtung nach Anspruch 2, **dadurch gekennzeichnet, dass** die Steuereinheit (30) konfiguriert ist, dass der dritte Grenzwert (G3) eine Konstante ist.

4. Blutbehandlungsvorrichtung nach zumindest einem der vorhergehenden Ansprüche, **dadurch gekennzeichnet, dass** die Steuereinheit (30) konfiguriert ist, dass im Bypass-Betrieb eine Substitutionsrate einer Prädilution kleiner als ein Substitutionsgrenzwert (S1) ist.

5. Blutbehandlungsvorrichtung nach zumindest einem der vorhergehenden Ansprüche, **dadurch gekennzeichnet, dass** die Steuereinheit (30) konfiguriert ist, dass im Bypass-Betrieb ein Dialysierflüssigkeitsfluss durch den Dialysator kleiner als ein Dialysierflüssigkeitsdialysatorfluss-Grenzwert (D1) ist.

6. Blutbehandlungsvorrichtung nach zumindest einem der vorhergehenden Ansprüche, **dadurch gekennzeichnet, dass** die Steuereinheit (30) konfiguriert ist, einen Alarm auszulösen und/oder eine Blutpumpe zu stoppen, wenn die zweite Blutkonzentration (B2) den dritten Grenzwert (G3) überschreitet.

7. Blutbehandlungsvorrichtung nach zumindest einem der vorhergehenden Ansprüche, **dadurch gekennzeichnet, dass** die Steuereinheit (30) konfiguriert ist, dass die Fließgeschwindigkeit (Q1) durch eine Pumpeinrichtung in der Dialysierflüssigkeitszuführleitung (15) vorgegeben wird.

8. Blutbehandlungsvorrichtung nach zumindest einem der vorhergehenden Ansprüche, **dadurch gekennzeichnet, dass** die Steuereinheit (30) konfiguriert ist, dass die Fließgeschwindigkeit (Q1) durch eine Bilanziereinrichtung (27) vorgegeben wird.

## Claims

1. A blood treatment device for extracorporeal blood treatment, in particular for haemodiafiltration, with a control unit (30) for detecting an overshoot of a first limit value (G1) of a first blood concentration (B1) in a first portion (17a) of a dialysate discharge line (17) downstream of a dialysate chamber (7) of a dialyser (4) of the blood treatment device and upstream of a node point (110) at which a bypass line (100) bypassing the dialyser (4) leads into the dialysate discharge line (17), wherein the bypass line (100) branches off, upstream of the dialysate chamber (7), from a dialysate supply line (15) suitable for supplying dialysate from a dialysate source (16) to the dialysate chamber (7),
wherein a blood leak detector (BS) is provided in a second portion (17b) of the dialysate discharge line (17) downstream of the node point (110) and is suitable for measuring a second blood concentration (B2) in the second portion (17b) downstream of the node point (110), wherein the control unit is configured to detect an overshoot of a second limit value (G2) of the second blood concentration (B2), wherein the second limit value (G2) decreases with rising flow rate (Q1) in the dialysate supply line (15) before the branch point of the bypass line (100), and wherein the control unit (30) is configured, in the event of bypass operation, in which case dialysate is guided from the dialysate source (16) via the dialysate supply line (15), the bypass line (100), and the second portion (17b) of the dialysate discharge line (17) to an outflow (18), to consider the first limit value (G1) as having been overshot when the second blood concentration (B2) exceeds a third limit value (G3), which is lower than the second limit value (G2) at the flow rate (Q1).

2. The blood treatment device according to claim 1, **characterised in that** the control unit (30) is configured in such a way that the third limit value (G3) is independent of the flow rate (Q1).

3. The blood treatment device according to claim 2, **characterised in that** the control unit (30) is configured in such a way that the third limit value (G3) is a constant.

4. The blood treatment device according to at least one of the preceding claims, **characterised in that** the control unit (30) is configured in such a way that in bypass operation a substitution rate of a pre-dilution is lower than a substitution limit value (S1) .

5. The blood treatment device according to at least one of the preceding claims, **characterised in that** the control unit (30) is configured in such a way that in bypass operation a dialysate flow through the dialyser is below a dialysate dialyser flow limit value (D1).

6. The blood treatment device according to at least one of the preceding claims, **characterised in that** the control unit (30) is configured to trigger an alarm and/or to stop a blood pump when the second blood concentration (B2) exceeds the third limit value (G3).

7. The blood treatment device according to at least one of the preceding claims, **characterised in that** the control unit (30) is configured in such a way that the flow rate (Q1) is predefined by a pump device in the dialysate supply line (15).

8. The blood treatment device according to at least one of the preceding claims, **characterised in that** the control unit (30) is configured in such a way that the flow rate (Q1) is predefined by a balancing device (27).

## Revendications

1. Dispositif de traitement sanguin pour le traitement sanguin extracorporel, en particulier pour l'hémodiafiltration, comportant une unité de commande (30) pour la détection d'un dépassement d'une première valeur limite (G1) d'une première concentration sanguine (B1) dans une première section (17a) d'une conduite d'évacuation de liquide de dialyse (17) en aval d'une chambre à liquide de dialyse (7) d'un dialyseur (4) du dispositif de traitement sanguin et en amont d'un point nodal (110) au niveau duquel une conduite de dérivation (100) contournant le dialyseur (4) débouche dans la conduite d'évacuation de liquide de dialyse (17), la conduite de dérivation (100) bifurquant en amont de la chambre à liquide de dialyse (7) d'une conduite d'alimentation en liquide de dialyse (15) qui est apte à acheminer du liquide de dialyse à la chambre à liquide de dialyse (7) depuis une source de liquide de dialyse (16),
étant prévu, dans une seconde section (17b) de la conduite d'évacuation de liquide de dialyse (17) en amont du point nodal (110), un détecteur de fuites de sang (BS) qui est apte à mesurer une seconde concentration sanguine (B2) dans la seconde section (17b) en aval du point nodal (110), l'unité de commande étant conçue pour détecter un dépassement d'une deuxième valeur limite (G2) de la seconde concentration sanguine (B2), la deuxième valeur limite (G2) diminuant au fur et à mesure de l'augmentation de la vitesse d'écoulement (Q1) dans la conduite d'alimentation en liquide de dialyse (15) en amont de la bifurcation de la conduite de dérivation (100) et l'unité de commande (30) étant conçue pour, dans un mode de dérivation dans lequel du liquide de dialyse est conduit depuis la source de liquide de dialyse (16) en passant par la conduite d'alimentation en liquide de dialyse (15), la conduite de dérivation (100) et la seconde section (17b) de la conduite d'évacuation de liquide de dialyse (17) vers une évacuation (18), considérer la première valeur limite (G1) comme dépassée si la seconde concentration sanguine (B2) dépasse une troisième valeur limite (G3) qui est plus basse que la deuxième valeur limite (G2) à la vitesse d'écoulement (Q1).

2. Dispositif de traitement sanguin selon la revendication 1, **caractérisé en ce que** l'unité de commande (30) est conçue pour que la troisième valeur limite (G3) soit indépendante de la vitesse d'écoulement (Q1).

3. Dispositif de traitement sanguin selon la revendication 2, **caractérisé en ce que** l'unité de commande (30) est conçue pour que la troisième valeur limite (G3) soit une constante.

4. Dispositif de traitement sanguin selon au moins une des revendications précédentes, **caractérisé en ce que** l'unité de commande (30) est conçue pour que, en mode de dérivation, un taux de substitution d'une pré-dilution soit inférieur à une valeur limite de substitution (S1).

5. Dispositif de traitement sanguin selon au moins une des revendications précédentes, **caractérisé en ce que** l'unité de commande (30) est conçue pour que, en mode de dérivation, un flux de liquide de dialyse à travers le dialyseur soit inférieur à une valeur limite de flux dialyseur de liquide de dialyse (D1).

6. Dispositif de traitement sanguin selon au moins une des revendications précédentes, **caractérisé en ce que** l'unité de commande (30) est conçue pour déclencher une alarme et/ou stopper une pompe hémostatique lorsque la seconde concentration sanguine (B2) dépasse la troisième valeur limite (G3).

7. Dispositif de traitement sanguin selon au moins une des revendications précédentes, **caractérisé en ce que** l'unité de commande (30) est conçue pour que la vitesse d'écoulement (Q1) soit prédéfinie par un système de pompage dans la conduite d'alimentation en liquide de dialyse (15).

8. Dispositif de traitement sanguin selon au moins une des revendications précédentes, **caractérisé en ce que** l'unité de commande (30) est conçue pour que la vitesse d'écoulement (Q1) soit prédéfinie par un système d'équilibrage (27).
